# EUROPÄISCHE PATENTANMELDUNG

(11) **EP 3 424 420 A1**
(43) Veröffentlichungstag der Anmeldung: **09.01.2019**
(21) Anmeldenummer: 17180288.7
(22) Anmeldetag: 07.07.2017
(51) Int. Cl.: A61B 5/055, A61B 5/06

(54) **VERFAHREN ZU EINEM UNTERSTÜTZEN EINES BENUTZERS BEI EINEM POSITIONIEREN EINER ZUBEHÖREINHEIT FÜR EINE MAGNETRESONANZUNTERSUCHUNG AN EINEM UNTERSUCHUNGSOBJEKT UND EINE MAGNETRESONANZVORRICHTUNG**

(71) Anmelder: Siemens Healthcare GmbH, 91052 Erlangen (DE)
(72) Erfinder: Faigle, Christine, 91054 Erlangen (DE); Heismann, Björn, 91058 Erlangen (DE); Quick, Silke, 45239 Essen (DE); Rothgang, Eva, 90571 Schwaig bei Nürnberg (DE)

(57) **Zusammenfassung**

Die Erfindung geht aus von einem Verfahren zu einem Unterstützen eines Benutzers bei einem Positionieren einer Zubehöreinheit für eine Magnetresonanzuntersuchung an einem Untersuchungsobjekt mit den folgenden Schritten:
- Erfassen einer Ist-Position der Zubehöreinheit mittels einer Erfassungseinheit,
- Ermitteln einer Soll-Position der Zubehöreinheit mittels einer Ermittlungseinheit,
- Vergleichen der Ist-Position der Zubehöreinheit mit der Soll-Position der Zubehöreinheit hinsichtlich einer Abweichung der Ist-Position der Zubehöreinheit von der Soll-Position der Zubehöreinheit,
- Generieren einer ersten Ausgabeinformation anhand des Vergleichs der Ist-Position der Zubehöreinheit mit der Soll-Position der Zubehöreinheit und
- Ausgabe der ersten Ausgabeinformation an einen Benutzer mittels einer Ausgabeeinheit.

## Beschreibung

Die vorliegende Erfindung betrifft ein Verfahren zu einem Unterstützen eines Benutzers bei einem Positionieren einer Zubehöreinheit für eine Magnetresonanzuntersuchung an einem Untersuchungsobjekt. Des Weiteren umfasst die vorliegende Erfindung eine Magnetresonanzvorrichtung, die zu einer Ausführung des Verfahrens zu einem Unterstützen eines Benutzers bei einem Positionieren einer Zubehöreinheit für eine Magnetresonanzuntersuchung an einem Untersuchungsobjekt ausgelegt ist. Die Erfindung umfasst weiterhin ein Computerprogrammprodukt, welches ein Programm zu einem Ausführen des Verfahrens zu einem Unterstützen eines Benutzers bei einem Positionieren einer Zubehöreinheit für eine Magnetresonanzuntersuchung an einem Untersuchungsobjekt umfasst, sowie einen elektronisch lesbarer Datenträger, auf dem das Programm zum Ausführen des Verfahrens zu einem Unterstützen eines Benutzers bei einem Positionieren einer Zubehöreinheit für eine Magnetresonanzuntersuchung an einem Untersuchungsobjekt gespeichert ist.

Für eine Magnetresonanzuntersuchung sollten Zubehöreinheiten, wie beispielsweise lokale Hochfrequenzantenneneinheiten, die zu einem Empfang von Magnetresonanzsignalen ausgelegt sind, möglichst eng an einem Untersuchungsobjekt, beispielsweise um den zu untersuchenden Körperbereich des Patienten, anliegen. Falsch positionierte und/oder zu weit vom zu untersuchenden Körperbereich entfernt angeordnete Hochfrequenzantenneneinheiten können jedoch zu Bildartefakten in den erfassten Bilddaten oder auch zu einem hohen Signal-zu-Rausch-Verhältnis führen. Dies kann sogar dazu führen, dass die Magnetresonanzuntersuchung wiederholt werden muss, um sinnvolle und/oder auswertbare Bilddaten zu erhalten. Aufgrund der hohen Belastung für den Patienten und des erhöhten Zeitaufwands sind jedoch Wiederholungen von Magnetresonanzuntersuchungen unerwünscht.

Um jedoch die für die anstehende Magnetresonanzuntersuchung erforderliche lokale Hochfrequenzantenneneinheit korrekt am zu untersuchenden Körperbereich des Patienten zu positionieren, sind sehr gute und umfangreiche anatomische Kenntnisse des Benutzers erforderlich. Je nach Ausbildungsgrad des Benutzers sind derartige Kenntnisse oft nur unzureichend vorhanden.

DE 10 2009 004 448 B4 beschreibt ein Verfahren zu einem Erfassen einer Positionierungsinformation einer Hochfrequenzantenneneinheit bezüglich einer Magnetresonanzvorrichtung. Des Weiteren ist aus DE 10 2012 200 600 A1 eine Erkennung und Positionsbestimmung von lokalen Hochfrequenzantenneneinheiten mittels RFID bekannt.

Der vorliegenden Erfindung liegt insbesondere die Aufgabe zugrunde, einen Benutzer bei einer korrekten Positionierung einer Zubehöreinheit an einem Untersuchungsobjekt für eine Magnetresonanzuntersuchung zu unterstützen. Die Aufgabe wird durch die Merkmale der unabhängigen Ansprüche gelöst. Vorteilhafte Ausgestaltungen sind in den Unteransprüchen beschrieben.

Die Erfindung geht aus von einem Verfahren zu einem Unterstützen eines Benutzers bei einem Positionieren einer Zubehöreinheit für eine Magnetresonanzuntersuchung an einem Untersuchungsobjekt mit den folgenden Schritten:
- Erfassen einer Ist-Position der Zubehöreinheit mittels einer Erfassungseinheit,
- Ermitteln einer Soll-Position der Zubehöreinheit mittels einer Ermittlungseinheit,
- Vergleichen der Ist-Position der Zubehöreinheit mit der Soll-Position der Zubehöreinheit hinsichtlich einer Abweichung der Ist-Position der Zubehöreinheit von der Soll-Position der Zubehöreinheit,
- Generieren einer ersten Ausgabeinformation anhand des Vergleichs der Ist-Position mit der Soll-Position der Zubehöreinheit und
- Ausgabe der ersten Ausgabeinformation an einen Benutzer mittels einer Ausgabeeinheit.

Die Ist-Position der Zubehöreinheit umfasst bevorzugt eine aktuelle Position der Zubehöreinheit, die diese an dem Untersuchungsobjekt und/oder bezüglich des Untersuchungsobjekts einnimmt. Die Ist-Position kann dabei eine Position in zwei Dimensionen und/oder auch in drei Dimensionen als auch eine Orientierung der Zubehöreinheit an dem Untersuchungsobjekt, insbesondere am Patienten, und/oder bezüglich des Untersuchungsobjekts, insbesondere des Patienten, umfassen. Vorzugsweise umfasst die Erfassungseinheit eine separat zu einer Scannereinheit der Magnetresonanzvorrichtung ausgebildete Einheit. Beispielsweise kann die Erfassungseinheit eine Sensoreinheit und/oder eine Kameraeinheit umfassen. Alternativ oder zusätzlich kann es auch vorgesehen sein, dass die Erfassungseinheit von der Scannereinheit der Magnetresonanzvorrichtung umfasst ist. Hierbei kann die Ist-Position der Zubehöreinheit anhand von Markierungselementen, die auf der Zubehöreinheit angeordnet sind, in erfassten Magnetresonanzbilddaten erfasst und/oder ermittelt werden. Alternativ oder zusätzlich zu einer Erfassung der Ist-Position mittels einer Sensoreinheit und/oder einer Kameraeinheit kann die Ist-Position der Zubehöreinheit auch mittels eines Triangulationsverfahrens bestimmt werden. Die Ist-Position der Zubehöreinheit kann bevorzugt mit einer Genauigkeit von maximal einigen mm bestimmt werden.

Die Zubehöreinheit kann bevorzugt eine lokale Hochfrequenzantenneneinheit umfassen, da diese für eine Erfassung von Magnetresonanzsignalen direkt um den zu untersuchenden Körperbereich des Patienten angeordnet werden sollen. Alternativ oder zusätzlich kann die Zubehöreinheit auch eine Infusionseinheit und/oder eine Lagerungseinheit, wie beispielsweise ein Lagerungskissen, und/oder ein Atemgurt und/oder weitere, dem Fachmann als sinnvoll erscheinende Zubehöreinheiten, die für eine Magnetresonanzuntersuchung erforderlich sind, umfassen.

Das Untersuchungsobjekt umfasst bevorzugt einen Patienten, insbesondere einen menschlichen und/oder tierischen Patienten. Alternativ oder zusätzlich kann das Untersuchungsobjekt auch ein Magnetresonanz-Phantom umfassen.

Die Soll-Position umfasst bevorzugt eine ideale Position der Zubehöreinheit, die die Zubehöreinheit für die anstehende Magnetresonanzuntersuchung an dem Untersuchungsobjekt, insbesondere an einem zu untersuchenden Bereich des Untersuchungsobjekts, haben sollte. Die Soll-Position kann dabei anhand von Untersuchungsinformationen eines Patienten ermittelt werden, wobei die Untersuchungsinformation ein zu untersuchender Körperbereich eines Patienten umfasst. Die Untersuchungsinformationen können dabei in einer Datenbank hinterlegt sein oder von einem Benutzer eingegeben werden. Zudem kann die Soll-Position auch anhand von erfassten Magnetresonanzbilddaten des Untersuchungsobjekts und/oder anhand von erfassten Positionierungsdaten, die mittels einer Kamera erfasst werden, des Untersuchungsobjekts ermittelt werden. Beispielsweise kann hierbei anhand einer erfassten Größe des Patienten und/oder einer Position eines zu untersuchenden Bereichs des Untersuchungsobjekts die Soll-Position der Zubehöreinheit ermittelt werden. Zudem kann die Soll-Position der Zubehöreinheit auch anhand eines Fixierungszustands und/oder Fixierungsstatus der Zubehöreinheit an einer Patientenlagerungsvorrichtung der Magnetresonanzvorrichtung erfasst werden.

Die Ermittlungseinheit ist bevorzugt von der Magnetresonanzvorrichtung umfasst. Die Ermittlungseinheit kann dabei einen Prozessor umfassen, der zur Ermittlung der Soll-Position der Zubehöreinheit ausgelegt ist. Für die Ermittlung der Soll-Position der Zubehöreinheit kann die Ermittlungseinheit auch eine Software und/oder Computerprogramme umfassen und/oder auf diese zugreifen, die in einer Speichereinheit und/oder einem Datenträger hinterlegt sind und die bei einer Ausführung zur Ermittlung der Soll-Position der Zubehöreinheit beitragen.

Mittels der Ermittlungseinheit kann die Ist-Position der Zubehöreinheit mit der Soll-Position der Zubehöreinheit verglichen werden. Für den Vergleich der Ist-Position der Zubehöreinheit mit der Soll-Position der Zubehöreinheit kann die Ermittlungseinheit auch eine Software und/oder Computerprogramme umfassen und/oder auf diese zugreifen, die in einer Speichereinheit und/oder einem Datenträger hinterlegt sind und die bei einer Ausführung den Vergleich der Ist-Position der Zubehöreinheit mit der Soll-Position der Zubehöreinheit ausführen. Die Abweichung der Ist-Position der Zubehöreinheit von der Soll-Position der Zubehöreinheit umfasst dabei eine Differenz und/oder einen Unterschied zwischen der Ist-Position der Zubehöreinheit und der Soll-Position der Zubehöreinheit.

Die erste Ausgabeinformation kann eine optische erste Ausgabeinformation und/oder eine akustische erste Ausgabeinformation umfassen. Die Ausgabeeinheit ist bevorzugt von der Magnetresonanzvorrichtung und/oder der Zubehöreinheit umfasst und kann eine optische Ausgabeeinheit, beispielsweise ein Monitor und/oder ein Display, und/oder eine akustische Ausgabeeinheit, beispielsweise Lautsprecher, umfassen. Zudem kann die Ausgabeeinheit direkt an einer Scannereinheit der Magnetresonanzvorrichtung angeordnet sein, so dass eine direkte Informationsübermittlung an den Benutzer während eines Positionierens der Zubehöreinheit an dem Untersuchungsobjekt ermöglicht werden kann. Auch eine direkte Anordnung der Ausgabeeinheit an der Zubehöreinheit kann vorgesehen sein, wodurch ebenfalls eine direkte Informationsübermittlung an den Benutzer während eines Positionierens der Zubehöreinheit an dem Untersuchungsobjekt ermöglicht werden kann. Vorzugsweise zeigt die erste Ausgabeinformation einem Benutzer, beispielsweise einem die anstehende Magnetresonanzuntersuchung an dem Untersuchungsobjekt betreuenden und/oder vorbereitenden medizinischen Bedienpersonal, an, wie groß eine Abweichung der Ist-Position der Zubehöreinheit von der Soll-Position ist. Die erste Ausgabeinformation kann aber auch die Soll-Position der Zubehöreinheit anzeigen und/oder ausgeben.

Die Erfindung weist den Vorteil auf, dass eine Zubehöreinheit, wie beispielsweise eine lokale Hochfrequenzantenneneinheit, auch von einem weniger gut geschulten Personal oder einem unerfahrenen Personal korrekt an dem Untersuchungsobjekt, insbesondere an einem zu untersuchenden Bereich des Untersuchungsobjekts, positioniert werden kann. Des Weiteren kann auch eine Lage und/oder Position und/oder Orientierung der Zubehöreinheit individuell für die anstehende Magnetresonanzuntersuchung an dem Untersuchungsobjekt, insbesondere an dem Patienten, optimiert werden. Dies hat auch zur Folge, dass Magnetresonanzbilddaten mit einer hohen Qualität erfasst und/oder akquiriert werden können und damit Magnetresonanzbilddaten mit einer hohen Aussagekraft bereitgestellt werden können. Unerwünschte Wiederholungen von Magnetresonanzuntersuchungen können derart vorteilhaft verhindert werden.

Des Weiteren kann es in einer weiteren Ausgestaltung der Erfindung vorgesehen sein, dass das Verfahren zu einem Unterstützen eines Benutzers bei einem Positionieren einer Zubehöreinheit für eine Magnetresonanzuntersuchung an einem Untersuchungsobjekt die zusätzlichen Schritte umfasst:
- Ermitteln einer Positionskorrektur der Ist-Position der Zubehöreinheit, sofern eine Abweichung der Ist-Position der Zubehöreinheit von der Soll-Position der Zubehöreinheit vorliegt, mittels der Ermittlungseinheit,
- Generieren einer zweiten Ausgabeinformation umfassend die Positionskorrektur der Ist-Position der Zubehöreinheit,
- Ausgabe der zweiten Ausgabeinformation an einen Benutzer mittels der Ausgabeeinheit.

Die zweite Ausgabeinformation kann zudem in einem einzigen Ausgabeschritt mit der ersten Ausgabeinformation mittels der Ausgabeeinheit an den Benutzer ausgegeben werden.

Vorzugsweise wird die Positionskorrektur anhand der ermittelten Abweichung zwischen der Ist-Position der Zubehöreinheit und der Soll-Position der Zubehöreinheit ermittelt und/oder bestimmt, so dass bei Berücksichtigung der Positionskorrektur der Ist-Position der Zubehöreinheit daraufhin die Ist-Position der Zubehöreinheit mit der Soll-Position der Zubehöreinheit übereinstimmt. Die zweite Ausgabeinformation umfassend die Positionskorrektur umfasst bevorzugt eine Handlungsanweisung für den Benutzer zur Korrekten Positionierung der Zubehöreinheit für die anstehende Magnetresonanzuntersuchung. Eine derartige Handlungsanweisung kann beispielsweise eine Anweisung zur Drehung und/oder Rotation und/oder Verschiebung der Zubehöreinheit an dem Untersuchungsobjekt umfassen.

Erfindungsgemäß kann es vorgesehen sein, dass die Zubehöreinheit eine an dem Untersuchungsobjekt anliegende Hochfrequenzantenneneinheit zur Erfassung von Magnetresonanzsignalen umfasst. Die Hochfrequenzantenneneinheit ist bevorzugt um einen zu untersuchenden Bereich des Untersuchungsobjekts angeordnet. Für eine Erfassung von qualitativ hochwertigen Bilddaten ist eine korrekte Position und/oder Orientierung einer lokalen Hochfrequenzantenneneinheit an dem Untersuchungsobjekt, insbesondere dem Patienten, erforderlich. Beispielsweise kann eine Knie-Hochfrequenzantenneneinheit für eine Knie-Untersuchung am Patienten verwendet werden und ist hierfür um das zu untersuchende Knie des Patienten angeordnet. Hierdurch können vorteilhaft lokale Hochfrequenzantenneneinheiten, deren Position und/oder Orientierung für einen Benutzer oft schwierig einzustellen sind, besonders einfach und schnell auf dem Untersuchungsobjekt positioniert werden.

Darüber hinaus kann es vorgesehen sein, dass das Erfassen der Ist-Position der Zubehöreinheit ein Sensor-basiertes Erfassen der Ist-Position der Zubehöreinheit umfasst, wobei das Sensor-basierte Erfassen der Ist-Position der Zubehöreinheit anhand von Referenzpunkten des Untersuchungsobjekts und/oder anhand von Referenzpunkten der Magnetresonanzvorrichtung erfolgt. Die Referenzpunkte können bevorzugte Körpermerkmale des Untersuchungsobjekts, insbesondere des Patienten, umfassen, wie beispielsweise Gelenkpunkte des Patienten. Die Referenzpunkte können aber auch bevorzugte Positionen und/oder Punkte des Magnetresonanzgeräts umfassen, wie beispielsweise ein Randbereich eines Patientenaufnahmebereichs und/oder ein Randbereich eines Patiententisches einer Patientenlagerungsvorrichtung. Zudem können die Referenzpunkte des Untersuchungsobjekts und/oder die Referenzpunkte der Magnetresonanzvorrichtung auch mittels Markierungselementen versehen werden, so dass eine eindeutige Zuordnung der jeweiligen Referenzpunkte in den erfassten Aufnahmen zur Erfassung der Ist-Position der Zubehöreinheit erfolgen kann. Das Sensor-basierte Erfassen erfolgt bevorzugt mittels einer Sensoreinheit, wie beispielsweise einer Kameraeinheit, insbesondere einer 2D-Kameraeinheit oder einer 3D-Kameraeinheit. Ein Vorteil hiervon ist, dass ein einfaches und schnelles Erfassen der Ist-Position der Zubehöreinheit für eine Unterstützung des Benutzers zur Verfügung gestellt werden kann.

In einer weiteren Ausgestaltung der Erfindung kann es vorgesehen sein, dass das Erfassen der Ist-Position der Zubehöreinheit ein Erfassen einer Orientierung der Zubehöreinheit im Raum umfasst. Hierdurch kann vorteilhaft eine dreidimensionale Ausrichtung und/oder Lage der Zubehöreinheit, beispielsweise eine dreidimensionale Ausrichtung und/oder Lage einer lokalen Hochfrequenzantenneneinheit, erfasst werden. Dies ermöglicht zudem eine besonders exakte Positionierung und/der Ausrichtung der Zubehöreinheit an dem Untersuchungsobjekt.

Darüber hinaus kann es vorgesehen sein, dass das Erfassen der Ist-Position der Zubehöreinheit ein Erfassen eines Fixierungsstatus der Zubehöreinheit umfasst. Der Fixierungsstatus der Zubehöreinheit umfasst bevorzugt einen aktuellen Fixierungszustand der Zubehöreinheit. Beispielsweise kann der Fixierungsstatus der Zubehöreinheit eine Information umfassen, ob die Zubehöreinheit fixiert ist, wie beispielsweise mittels einer Fixierungseinheit an insbesondere eine Patientenlagerungsvorrichtung der Magnetresonanzvorrichtung, oder auch ob die Zubehöreinheit nicht fixiert ist. Vorzugsweise weist hierzu die Fixierungseinheit exakt definierte Fixierungspositionen auf, so dass besonders vorteilhaft in einem fixierten Zustand der Zubehöreinheit auch auf die Ist-Position der Zubehöreinheit geschlossen und/oder eine Positionsinformation der Zubehöreinheit zur Ermittlung der Ist-Position bereitgestellt werden kann. Der Fixierungsstatus für eine Fixierungsposition der Fixierungseinheit kann beispielsweise elektronisch mittels eines Steckkontakts der Fixiereinheit erfasst werden. Dies ermöglicht es, dass bereits vorliegende Positionsinformationen bezüglich der Position der Zubehöreinheit zur Bestimmung der Ist-Position der Zubehöreinheit verwendet werden können.

Ferner kann es vorgesehen sein, dass das Ermitteln der Soll-Position der Zubehöreinheit ein Ermitteln einer Untersuchungsregion des Untersuchungsobjekts umfasst. Die Untersuchungsregion des Untersuchungsobjekts umfasst bevorzugt einen zu untersuchenden Bereich des Untersuchungsobjekts. Hierdurch kann eine besonders individuelle, auf das Untersuchungsobjekt, insbesondere auf die Position und/der Lage der Untersuchungsregion, abgestimmte Soll-Position der Zubehöreinheit ermittelt werden. Vorteilhaft können derart auch anatomische Eigenschaften des Untersuchungsobjekts, insbesondere eines Patienten, bei der Ermittlung und/oder der Bestimmung der Soll-Position der Zubehöreinheit berücksichtigt werden. Beispielsweise kann eine absolute Position eine zu untersuchenden Organs bezüglich eines Referenzpunkts der Magnetresonanzvorrichtung von einer Größe des Patienten abhängen.

Darüber hinaus kann es vorgesehen sein, dass das Ermitteln der Soll-Position der Zubehöreinheit ein Ermitteln anhand einer Übersichtsmessung des Untersuchungsobjekts umfasst. Die Übersichtsmessung, insbesondere eine Localizer-Messung, wird bevorzugt vor der anstehenden Magnetresonanzuntersuchung an einem Patienten durchgeführt, um anhand der Übersichtsmessung die anstehende Magnetresonanzuntersuchung zu planen. Die Übersichtsmessung, insbesondere die Localizer-Messung, wird bevorzugt mit einer geringeren Auflösung in den Bilddaten erfasst als eine Auflösung von Bilddaten der anstehenden Magnetresonanzuntersuchung. Vorzugsweise umfasst die Übersichtsmessung Bilddaten von dem zu untersuchenden Bereich des Untersuchungsobjekts, wie beispielsweise einen zu untersuchenden Organbereich des Patienten. Zudem kann die Übersichtsmessung auch Bilddaten von dem gesamten Untersuchungsobjekt, wie beispielsweise den gesamten Patienten, umfassen. Hierdurch können vorteilhaft anhand von ohnehin existierenden und/der zu erfassenden Übersichtsdaten besonders Zeitsparend Daten zur Ermittlung und/oder Bestimmung der Soll-Position der Zubehöreinheit bereitgestellt werden. Zudem kann derart auf zusätzliche Messungen zur Erfassung von Daten zu Ermittlung und/oder Bestimmung der Soll-Position der Zubehöreinheit vorteilhaft verzichtet werden.

In einer vorteilhaften Weiterbildung der Erfindung kann es vorgesehen sein, dass zur Bestimmung der Soll-Position der Zubehöreinheit ein Model des Untersuchungsobjekts erstellt wird. Das Model umfasst bevorzugt ein künstlich generiertes Bild oder eine künstlich generierte Figur, beispielsweise eine Grafikfigur und/oder einer 3D-Figur , die in virtuellen Umgebungen stellvertretend für das Untersuchungsobjekt steht, wie beispielsweise ein Avatar. Vorzugsweise wird das Model mittels der Ermittlungseinheit und/oder einer weiteren Einheit erstellt und/oder generiert, wobei zur Ermittlung akquirierte Daten einer Übersichtmessung zugrunde liegen können, so dass ein möglichst große Übereinstimmung zwischen dem Model und dem Untersuchungsobjekt vorliegt. Alternativ oder zusätzlich kann das Model anhand von bereits vorliegenden Bilddaten des Untersuchungsobjekts und/oder auch anhand von Kameradaten erstellt und/oder generiert werden. Zur Bestimmung und/der Ermittlung des Models kann das Model an die Bilddaten und/der die Übersichtsdaten und/der die Kameradaten angefittet werden. Die Ermittlungseinheit und/oder die weitere Einheit kann hierzu bevorzugt eine Software und/oder Computerprogramme umfassen und/oder auf diese zugreifen, die in einer Speichereinheit und/oder einem Datenträger hinterlegt sind und die bei einer Ausführung die Generierung und/oder Erstellung eines Models des Untersuchungsobjekts ausführen. Diese Ausgestaltung der Erfindung weist den Vorteil auf, dass die Position der Zubehöreinheit individuell an das Untersuchungsobjekt, insbesondere an beispielsweise anatomische Eigenschaften des Untersuchungsobjekts, angepasst oder optimiert werden kann. Dies ermöglicht auch einem weniger geschulten medizinischen Bedienpersonal ein einfaches Positionieren und/oder Anlegen der Zubehöreinheit an dem Untersuchungsobjekt.

Darüber hinaus wird vorgeschlagen, dass die erste Ausgabeinformation eine Positionierungsinformation für die Soll-Position der Zubehöreinheit umfasst. Hierdurch kann eine vorteilhafte Darstellung der Soll-Position für den Benutzer, insbesondere einem medizinischen Bedienpersonal, zur Positionierung der Zubehöreinheit erreicht werden. Insbesondere kann der Benutzer direkt eine korrekte Position der Zubehöreinheit anhand der dargestellten Positionierungsinformation erkennen.

In einer vorteilhaften Weiterbildung der Erfindung kann es vorgesehen sein, dass eine weitere Ausgabeinformation generiert wird, sobald die Ist-Position der Zubehöreinheit mit der Soll-Position der Zubehöreinheit übereinstimmt. Hierdurch kann der Vorteil erreicht werden, dass der Benutzer ein direktes Feedback erhält oder direkt informiert wird, sobald die Ist-Position der Zubehöreinheit mit der Soll-Position der Zubehöreinheit übereinstimmt. Vorzugsweise wird die weitere Ausgabeinformation direkt nach dem Generieren der weiteren Ausgabeinformation mittels der Ausgabeeinheit ausgeben. Bevorzugterweise kann die weitere Ausgabeinformation auch eine Handlungsanweisung an den Benutzer umfassen, wie beispielsweise dass die Zubehöreinheit in der aktuellen Position, die bevorzugt die Soll-Position der Zubehöreinheit ist, fixiert werden kann und/oder sollte. Zudem kann die weitere Ausgabeinformation auch nur ein Aufleuchten einer Kontrollleuchte umfassen, das dem Benutzer signalisiert, dass die Zubehöreinheit in ihrer Soll-Position angeordnet ist. Hierdurch kann ein Zeit sparendes und einfaches Positionieren der Zubehöreinheit an dem Untersuchungsobjekt vorteilhaft erreicht werden.

Des Weiteren geht die Erfindung aus von einer Magnetresonanzvorrichtung mit einer Scannereinheit, einer Erfassungseinheit zu einem Erfassen einer Ist-Position einer Zubehöreinheit, einer Ermittlungseinheit zu einem Ermitteln einer Soll-Position der Zubehöreinheit, einer Ausgabeeinheit und einer Zubehöreinheit, wobei die Magnetresonanzvorrichtung zu einem Ausführen des Verfahrens zu einem Unterstützen eines Benutzers bei einem Positionieren einer Zubehöreinheit für eine Magnetresonanzuntersuchung an einem Untersuchungsobjekt ausgelegt ist.

Eine derartig ausgebildete Magnetresonanzvorrichtung weist den Vorteil auf, dass eine Zubehöreinheit, wie beispielsweise eine lokale Hochfrequenzantenneneinheit, auch von einem weniger gut geschulten Personal oder einem unerfahrenen Personal korrekt an dem Untersuchungsobjekt, insbesondere an einem zu untersuchenden Bereich des Untersuchungsobjekts, beispielweise einem Patienten, positioniert werden kann. Des Weiteren kann auch eine Lage und/oder Position und/oder Orientierung der Zubehöreinheit individuell für die anstehende Magnetresonanzuntersuchung an dem Untersuchungsobjekt, insbesondere an dem Patienten, optimiert werden. Dies hat auch zur Folge, dass Magnetresonanzbilddaten mit einer hohen Qualität erfasst und/oder akquiriert werden können und damit Magnetresonanzbilddaten mit einer hohen Aussagekraft bereitgestellt werden können. Unerwünschte Wiederholungen von Magnetresonanzuntersuchungen können derart vorteilhaft verhindert werden.

Die Vorteile der erfindungsgemäßen Magnetresonanzvorrichtung entsprechen im Wesentlichen den Vorteilen des erfindungsgemäßen Verfahrens zu einem Unterstützen eines Benutzers bei einem Positionieren einer Zubehöreinheit für eine Magnetresonanzuntersuchung an einem Untersuchungsobjekt, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

In einer weiteren Ausgestaltung der Magnetresonanzvorrichtung kann es vorgesehen sein, dass die Erfassungseinheit eine Sensoreinheit umfasst. Dies ermöglicht eine einfache und direkte Erfassung der Ist-Position der Zubehöreinheit, insbesondere einer aktuellen Ist-Position der Zubehöreinheit, an dem Untersuchungsobjekt. Die Sensoreinheit kann dabei eine Kameraeinheit, beispielsweise eine 2D-Kameraeinheit und besonders vorteilhaft eine 3D-Kameraeinheit, umfassen. Die Kameraeinheit ist bevorzugt derart angeordnet, dass ein Vorbereitungsbereich zur Vorbereitung des Untersuchungsobjekts, insbesondere der Patienten, in einem Erfassungsbereich der Kameraeinheit angeordnet ist. Vorzugsweise ist hierbei das Untersuchungsobjekt auf einer Patientenlagerungsvorrichtung liegend direkt vor der Magnetresonanzvorrichtung positioniert.

Alternativ oder zusätzlich kann die Erfassungseinheit, insbesondere die Sensoreinheit, auch einen Rotationssensoreinheit umfassen. Dies ermöglicht eine besonders exakte Erfassung einer Orientierung und/oder Ausrichtung der Zubehöreinheit an dem Untersuchungsobjekt. Die Rotationssensoreinheit ist bevorzugt an der Zubehöreinheit und/oder innerhalb der Zubehöreinheit angeordnet, so dass eine Orientierung und/oder Lage der Zubehöreinheit direkt erfasst werden kann. Die Rotationssensoreinheit kann dabei einen Gyroskop-Sensor umfassen, wobei der Gyroskop-Sensor ein Beschleunigungssensor und/oder ein Lagesensor ist, der bereits auf geringe Beschleunigungen und/oder Drehbewegungen und/oder Lageänderungen, die auf den Gyroskop-Sensor einwirken, reagiert. Das Prinzip des Gyroskop-Sensors basiert insbesondere auf der Massenträgheit. Die von einem Gyroskop-Sensor erfassten Drehbewegungen und/oder Lageänderungen werden dabei in Form einer Spannungsänderung bezogen auf die Drehgeschwindigkeit ermittelt und ausgegeben und vorzugsweise von der Ermittlungseinheit ausgewertet.

Darüber hinaus kann es vorgesehen sein, dass die Erfassungseinheit eine Fixiereinheit umfasst. Mittels der Fixiereinheit kann ein aktueller Fixierstatus und/oder ein aktueller Fixierzustand der Zubehöreinheit ermittelt und/oder bestimmt werden und damit auch eine Positionsinformation zur Bestimmung der Ist-Position der Zubehöreinheit bereitgestellt werden. Die Fixiereinheit Die Fixiereinheit kann dabei bereits zumindest teilweise innerhalb der Zubehöreinheit integriert sein, wie beispielsweise ein in eine lokale Hochfrequenzantenneneinheit integrierter Steckkontakt, der bei Positionierung der lokalen Hochfrequenzantenneneinheit mit einer Patientenlagerungsvorrichtung der Magnetresonanzvorrichtung geschlossen wird.

In einer vorteilhaften Weiterbildung der Erfindung wird vorgeschlagen, dass die Zubehöreinheit eine an dem Untersuchungsobjekt anliegende Hochfrequenzantenneneinheit zur Erfassung von Magnetresonanzsignalen umfasst. Hierdurch können vorteilhaft lokale Hochfrequenzantenneneinheiten, deren Position und/oder Orientierung für einen Benutzer oft schwierig einzustellen sind, besonders einfach und schnell auf dem Untersuchungsobjekt positioniert werden. Für eine Erfassung von qualitativ hochwertigen Bilddaten ist eine korrekte Position und/oder Orientierung einer lokalen Hochfrequenzantenneneinheit an dem Untersuchungsobjekt, insbesondere dem Patienten, erforderlich, so dass bei einer Ausbildung der Zubehöreinheit als lokale Hochfrequenzantenneneinheit das erfindungsgemäße Verfahren besonders gewinnbringend eingesetzt werden kann.

Ferner kann es vorgesehen sein, dass die Ausgabeeinheit an der Scannereinheit und/oder der Zubehöreinheit angeordnet ist. Hierdurch kann dem Benutzer während einer Vorbereitung des Untersuchungsobjekts, insbesondere einem Positionieren der Zubehöreinheit an dem Patienten, direkt eine Ausgabeinformation, insbesondere die erste Ausgabeinformation oder auch die erste Ausgabeinformation zusammen mit weiteren Ausgabeinformationen, übermittelt und/oder ausgeben werden. Die Ausgabeeinheit kann dabei eine optische Ausgabeeinheit und/oder eine akustische Ausgabeeinheit umfassen. Beispielsweise kann die Ausgabeeinheit ein direkt an der Zubehöreinheit angeordneten Display und/oder LEDs aufweisen.

Des Weiteren geht die Erfindung aus von einem Computerprogrammprodukt, welches ein Programm umfasst und direkt in einem Speicher einer programmierbaren Steuereinheit einer Magnetresonanzvorrichtung ladbar ist, mit Programmmitteln, um ein Verfahren zu einem Unterstützen eines Benutzers bei einem Positionieren einer Zubehöreinheit für eine Magnetresonanzuntersuchung an einem Untersuchungsobjekt auszuführen, wenn das Programm in der Steuereinheit der Magnetresonanzvorrichtung ausgeführt wird. Dabei benötigt das Computerprogramm eventuell Programmmittel, z.B. Bibliotheken und Hilfsfunktionen, um die entsprechenden Ausführungsformen des Verfahrens zu realisieren. Das Computerprogramm kann dabei eine Software mit einen Quellcode, der noch compiliert und gebunden oder der nur interpretiert werden muss, oder einen ausführbaren Softwarecode umfassen, der zur Ausführung nur noch in eine entsprechende Recheneinheit zu laden ist.

Des Weiteren geht die Erfindung aus von einem computerlesbaren und/oder elektronisch lesbaren Datenträger, welcher ein Programm umfasst, das zu einer Ausführung eines Verfahrens zu einem Unterstützen eines Benutzers bei einem Positionieren einer Zubehöreinheit für eine Magnetresonanzuntersuchung an einem Untersuchungsobjekt vorgesehen ist.

Weitere Vorteile, Merkmale und Einzelheiten der Erfindung ergeben sich aus dem im Folgenden beschriebenen Ausführungsbeispiel sowie anhand der Zeichnungen.

Es zeigen:
- Fig. 1: eine erfindungsgemäße Magnetresonanzvorrichtung in einer schematischen Darstellung und
- Fig. 2: ein Ablaufdiagramm eines erfindungsgemäßen Verfahrens zu einem Unterstützen eines Benutzers bei einem Positionieren einer Zubehöreinheit für eine Magnetresonanzuntersuchung an einem Untersuchungsobjekt.

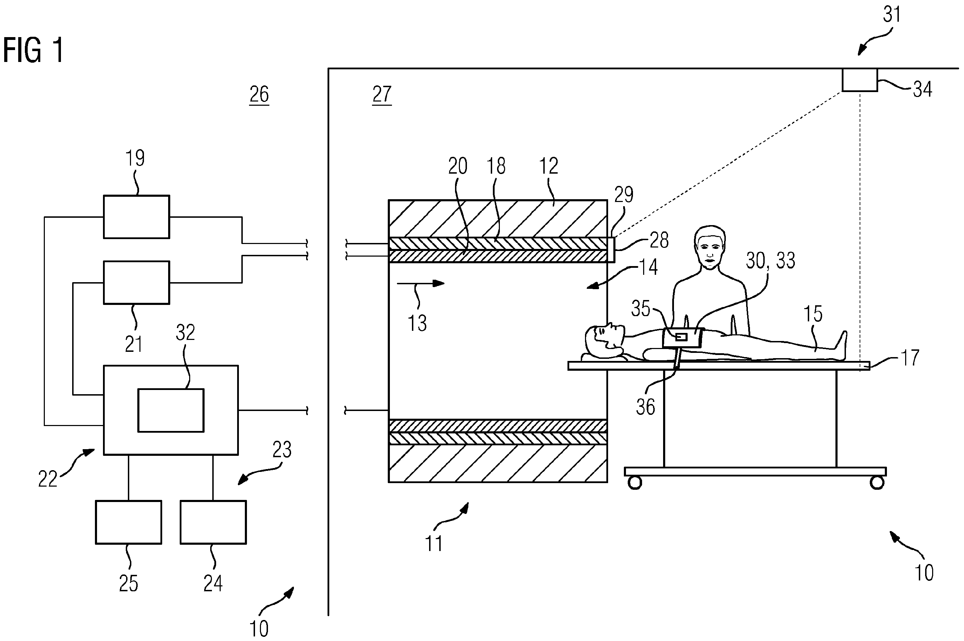

In der Fig. 1 ist eine Magnetresonanzvorrichtung 10 dargestellt. Die Magnetresonanzvorrichtung 10 umfasst eine Scannereinheit 11, die einen supraleitenden Hauptmagneten 12 zu einem Erzeugen eines starken und insbesondere konstanten Hauptmagnetfelds 13 umfasst. Zudem weist die Magnetresonanzvorrichtung 10 einen Patientenaufnahmebereich 14 auf zu einer Aufnahme eines Untersuchungsobjekts 15, insbesondere eines Patienten. Der Patientenaufnahmebereich 14 im vorliegenden Ausführungsbeispiel ist zylinderförmig ausgebildet und in einer Umfangsrichtung von der Scannereinheit 11 zylinderförmig umgeben. Grundsätzlich ist jedoch eine davon abweichende Ausbildung des Patientenaufnahmebereichs 14 jederzeit denkbar. Das Untersuchungsobjekt 15, beispielsweise der Patient, kann mittels einer Patientenlagerungsvorrichtung 16 der Magnetresonanzvorrichtung 10 in den Patientenaufnahmebereich 14 geschoben und/oder gefahren werden. Die Patientenlagerungsvorrichtung 16 weist hierzu einen innerhalb des Patientenaufnahmebereichs 14 bewegbar ausgestalteten Patiententisch 17 auf.

Die Scannereinheit 11 weist weiterhin eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 der Magnetresonanzvorrichtung 10 gesteuert. Die Scannereinheit 11 umfasst weiterhin eine Hochfrequenzantenneneinheit 20 zu einer Anregung einer Polarisation, die sich in dem von dem Hauptmagneten 12 erzeugten Hauptmagnetfeld 13 einstellt. Die Hochfrequenzantenneneinheit 20 wird von einer Hochfrequenzantennensteuereinheit 21 der Magnetresonanzvorrichtung 10 gesteuert und strahlt hochfrequente Magnetresonanzsequenzen in den Patientenaufnahmebereich 14 der Magnetresonanzvorrichtung 10 ein.

Zu einer Steuerung des Hauptmagneten 12, der Gradientensteuereinheit 19 und zur Steuerung der Hochfrequenzantennensteuereinheit 21 weist die Magnetresonanzvorrichtung 10 eine Steuereinheit 22 auf. Die Steuereinheit 22 steuert zentral die Magnetresonanzvorrichtung 10, wie beispielsweise das Durchführen einer vorbestimmten bildgebenden Gradientenechosequenz. Zudem umfasst die Steuereinheit 22 eine nicht näher dargestellte Auswerteeinheit zu einer Auswertung von medizinischen Bilddaten, die während der Magnetresonanzuntersuchung erfasst werden. Die Steuereinheit 22 weist eine Software und/oder Computerprogramme und/oder kann auf diese zugreifen auf, die von einem Prozessor der Steuereinheit 22 ausgeführt werden. Die Software und/oder Computerprogramme ist derart ausgestaltet, dass bei einem Ausführen der Software und/oder Computerprogramme eine Steuerung des Hauptmagneten, der Gradientensteuereinheit 19 und der Hochfrequenzantennensteuereinheit 21 erfolgt. Zudem kann die Steuereinheit auch eine Software und/oder Computerprogramme umfassen und/oder auf diese zugreifen, die bei einem Ausführen mittels des Prozessors der Steuereinheit zu einem Steuern und/oder Ausführen einer Magnetresonanzuntersuchung mittels der Magnetresonanzvorrichtung ausgelegt sind.

Des Weiteren umfasst die Magnetresonanzvorrichtung 10 eine erste Benutzerschnittstelle 23, die mit der Steuereinheit 22 verbunden ist. Steuerinformationen wie beispielsweise Bildgebungsparameter, sowie rekonstruierte Magnetresonanzbilder können auf einer Anzeigeeinheit 24, beispielsweise auf zumindest einem Monitor, der ersten Benutzerschnittstelle 23 für ein medizinisches Bedienpersonal angezeigt werden. Weiterhin weist die erste Benutzerschnittstelle 23 eine Eingabeeinheit 25 auf, mittels der Informationen und/oder Parameter während eines Messvorgangs von dem medizinischen Bedienpersonal eingegeben werden können. Die erste Benutzerschnittstelle 23 ist innerhalb eines Kontrollraums 26 angeordnet, wobei innerhalb des Kontrollraums 26 auch die Steuereinheit 22 angeordnet ist.

Die Scannereinheit 11 dagegen ist innerhalb eines Untersuchungsraums 27 angeordnet. Der Untersuchungsraum 27 ist getrennt von dem Kontrollraum 26 ausgebildet. Vorzugsweise ist die Untersuchungsraum 27 nach außen und innen abgeschirmt, um störende Einflüsse abzuschirmen, die sowohl eine Magnetresonanzuntersuchung beeinflussen können als auch von der Magnetresonanzvorrichtung 10, insbesondere der Scannereinheit 11, verursacht werden können. Beispielweise ist der Untersuchungsraum 27 sowohl hinsichtlich eines Magnetfelds als auch gegen elektromagnetische Strahlung, insbesondere Hochfrequenzstrahlung, abgeschirmt.

Die Magnetresonanzvorrichtung 10 weist eine zweite Benutzerschnittstelle 28 mit einer Ausgabeeinheit 29 auf. Die zweite Benutzerschnittstelle 28 ist innerhalb des Untersuchungsraums 27 angeordnet. Die Ausgabeeinheit 29 umfasst im vorliegenden Ausführungsbeispiel eine optische Ausgabeeinheit mit einem Display, das direkt an der Scannereinheit angeordnet ist. Zudem kann es auch sein, dass die Ausgabeeinheit 29 direkt an einer Zubehöreinheit 30, wie beispielsweise einer lokalen Hochfrequenzantenneneinheit, angeordnet ist. Vorzugsweise kann das Display abnehmbar an der Scannereinheit 11 angeordnet sein. Das Display kann dabei auch von einem Touch-Display gebildet sein. Alternativ hierzu kann die Ausgabeeinheit 29 auch richtungsanzeigende LED-Elemente umfassen, die direkt an der Zubehöreinheit 30, insbesondere der lokalen Hochfrequenzantenneneinheit, angeordnet sein können. Die Ausgabeeinheit 29 kann zudem auch eine akustische Ausgabeeinheit umfassen.

Die Magnetresonanzvorrichtung 20 weist weiterhin eine Erfassungseinheit 31, eine Ermittlungseinheit 32 und die Zubehöreinheit 30 auf. Die Zubehöreinheit 30 ist im vorliegenden Ausführungsbeispiel von einer lokalen Hochfrequenzantenneneinheit 33 gebildet. Mittels der lokalen Hochfrequenzantenneneinheit 33 können während der Magnetresonanzuntersuchung Magnetresonanzsignale erfasst werden. Hierzu wird die lokale Hochfrequenzantenneneinheit 33 an den zu untersuchenden Bereich des Untersuchungsobjekts 15, insbesondere des Patienten, angelegt. Das Anlegen der Zubehöreinheit 30, der lokalen Hochfrequenzantenneneinheit 33, erfolgt bevorzugt manuell durch den Benutzer, insbesondere das medizinisches Bedienpersonal.

Grundsätzlich kann in einer weiteren Ausgestaltung der Erfindung die Zubehöreinheit 30 auch ein Lagerungselement zur Lagerung des Untersuchungsobjekts 15, eine Infusionseinheit und/oder weitere, dem Fachmann als sinnvoll erscheinende Zubehöreinheiten 30 umfassen.

Zur Erfassung einer Ist-Position der Zubehöreinheit 30 ist die Erfassungseinheit 31 der Magnetresonanzvorrichtung 10 ausgebildet. Die Erfassungseinheit 31 ist innerhalb des Untersuchungsraums 27 angeordnet. Die Erfassungseinheit 31 weist dabei eine Sensoreinheit 34 auf. Die Sensoreinheit 34 umfasst im vorliegenden Ausführungsbeispiel eine Kameraeinheit. Die Kameraeinheit kann dabei von einer 2D-Kameraeinheit gebildet sein. Besonders vorteilhaft ist die Kameraeinheit jedoch von einer 3D-Kameraeinheit gebildet. Zur Erfassung der Ist-Position der Zubehöreinheit 30 ist die Kameraeinheit derart innerhalb des Untersuchungsraums 27 angeordnet, dass die Patientenlagerungsvorrichtung 16 zusammen mit dem Untersuchungsobjekt 15 während einer Vorbereitung einer Magnetresonanzuntersuchung innerhalb eines Erfassungsbereichs der Kameraeinheit angeordnet ist. Beispielsweise ist die Kameraeinheit hierzu an einer Raumdecke des Untersuchungsraums 27 angeordnet.

Die Erfassungseinheit 31 weist des Weiteren eine Rotationssensoreinheit 35. Die Rotationssensoreinheit 35 umfasst im vorliegenden Ausführungsbeispiel einen Gyroskop-Sensor, der an der Zubehöreinheit 30 oder innerhalb der Zubehöreinheit 30 angeordnet ist. Die Erfassungseinheit 31 umfasst zudem auch eine Fixiereinheit 36 zu einer Fixierung der Zubehöreinheit 30 an der Patientenlagerungsvorrichtung 16.

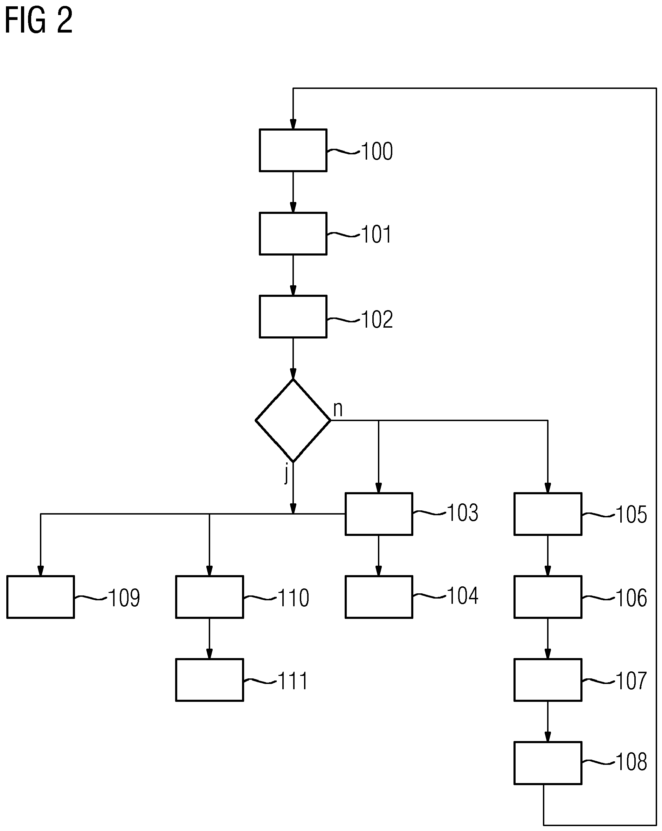

In Fig. 2 ist ein erfindungsgemäßes Verfahren zu einem Unterstützen eines Benutzers bei einem Positionieren der Zubehöreinheit 30 für eine Magnetresonanzuntersuchung an einem Untersuchungsobjekt 15 dargestellt. Das Verfahren wird automatisch und/oder selbsttätig von der Ermittlungseinheit 32 gesteuert. Zudem wird das Verfahren automatisch und/oder selbsttätig von der Ermittlungseinheit 32 zusammen mit der Erfassungseinheit 31 und der Ausgabeeinheit 29 ausgeführt. Die Erfassungseinheit 31 weist hierzu eine erforderliche Software und/oder Computerprogramme auf oder kann auf diese erforderliche Software und/oder Computerprogramme zugreifen, wobei die Software und/oder Computerprogramme in einer Speichereinheit und/oder einem elektronisch lesbaren Datenträger hinterlegt sind. Bei einer Ausführung der Software und/oder Computerprogramme mittels eines Prozessors der Ermittlungseinheit 31 führen die Software und/oder Computerprogramme ein Verfahren zu einem Unterstützen eines Benutzers bei einem Positionieren einer Zubehöreinheit 30 für eine Magnetresonanzuntersuchung an einem Untersuchungsobjekt 15 aus. Die Speichereinheit kann dabei von der Ermittlungseinheit 31 umfasst sein. Zudem kann die Speichereinheit eine externe Speichereinheit umfassen, auf die die Ermittlungseinheit 31 mittels eines Datennetzes zugreifen kann. Beispielsweise kann die Speichereinheit auch innerhalb einer Cloud angeordnet sein.

Das Untersuchungsobjekt 15, insbesondere der Patient befindet sich zu Beginn des Verfahrens bereits auf der Patientenlagerungsvorrichtung 15, insbesondere auf dem bewegbaren Patiententisch 16, angeordnet. Die Patientenlagerungsvorrichtung 16, insbesondere der bewegbare Patiententisch16, befindet sich zusammen mit dem Untersuchungsobjekt 15, insbesondere dem Patienten, innerhalb des Untersuchungsraums 27. Vorzugsweise ist die Patientenlagerungsvorrichtung 16, insbesondere der bewegbare Patiententisch 17, derart innerhalb des Untersuchungsraums 17 angeordnet, dass der Patiententisch 17 innerhalb der Erfassungsbereichs der Erfassungseinheit 31 angeordnet ist. Das Untersuchungsobjekt 15, insbesondere der Patient, ist dabei innerhalb des Erfassungsbereichs der Erfassungseinheit 31 angeordnet. Im vorliegenden Ausführungsbeispiel ist das Untersuchungsobjekt 15 beispielhaft von einem Patienten gebildet. Grundsätzlich ist auch eine Ausbildung des Untersuchungsobjekts 15 als Phantom und/oder weitere, dem Fachmann als sinnvoll erscheinende Ausbildungen des Untersuchungsobjekts 15 jederzeit denkbar.

Zunächst wird von dem Benutzer, insbesondere einem die Magnetresonanzuntersuchung betreuendem medizinischen Bedienpersonal, die Zubehöreinheit 30 an dem Patienten angelegt. In einem Verfahrensschritt 100 erfolgt daraufhin ein Erfassen einer Ist-Position der Zubehöreinheit 30, insbesondere einer aktuellen Position der Zubehöreinheit 30 an dem Patienten, mittels der Erfassungseinheit 31.

Das Erfassen der Ist-Position kann dabei mittels der Sensoreinheit 34, insbesondere der Kameraeinheit der Sensoreinheit 34, anhand eines Sensor-basierten Erfassens der Ist-Position der Zubehöreinheit 30 erfolgen. Vorzugsweise wird hierbei mittels der Kameraeinheit eine 2D-Position der Zubehöreinheit 30, also eine Position der Zubehöreinheit 30 in zwei Dimensionen, erfasst, insbesondere wenn die Kameraeinheit von der 2D-Kameraeinheit gebildet ist. Besonders vorteilhaft jedoch wird mittels der Kameraeinheit eine 3D-Position der Zubehöreinheit 30, also eine Position der Zubehöreinheit 30 in drei Dimensionen, erfasst, insbesondere wenn die Kameraeinheit von der 3D-Kameraeinheit gebildet ist.

Vorzugsweise erfolgt das Sensor-basierte Erfassen der Ist-Position der Zubehöreinheit 30 anhand und/oder bezüglich von Referenzpunkten. Die Referenzpunkte können dabei Referenzpunkte an dem Untersuchungsobjekt 15, insbesondere an dem Patienten, umfassen. Beispielsweise können die Referenzpunkte Gelenkpunkte des Patienten umfassen. Alternativ oder zusätzlich können die Referenzpunkte auch Referenzpunkte an der Magnetresonanzvorrichtung, wie beispielsweise Referenzpunkte an dem bewegbaren Patiententisch 17 oder Referenzpunkte in einem Öffnungsbereich des Patientenaufnahmebereichs 14 der Magnetresonanzvorrichtung 10, umfassen. Mittels der Kameraeinheit 34 kann somit eine Position der Zubehöreinheit 30 bezüglich dieser Referenzpunkte erfasst werden.

Das Erfassen der Ist-Position der Zubehöreinheit 30 kann auch mittels eines Triangulationsverfahrens erfolgen. Hierbei können auch die Kameradaten der Kameraeinheit 34 zur Erfassung der Ist-Position der Zubehöreinheit 30 verwendet werden. Zur Bestimmung der Ist-Position der Zubehöreinheit 30 mittels des Triangulationsverfahrens kann die Erfassungseinheit 31 auch eine Software und/oder Computerprogramme aufweisen, die dazu ausgebildet sind, mittels des Triangulationsverfahrens die Ist-Position der Zubehöreinheit 30 zu ermitteln. Zudem kann es auch vorgesehen sein, dass die Bestimmung der Ist-Position der Zubehöreinheit 30 mittels des Triangulationsverfahrens anhand von Kameradaten mittels der Ermittlungseinheit 31 erfolgt. Alternativ oder zusätzlich kann auch die Ermittlungseinheit 32 eine Software und/oder Computerprogramme umfassen, die dazu ausgebildet sind, mittels des Triangulationsverfahrens die Ist-Position der Zubehöreinheit 30 anhand der Kameradaten der Kameraeinheit zu ermitteln.

Zudem kann das Erfassen der Ist-Position auch mittels der Rotationssensoreinheit 35 erfolgen. Hierbei wird mittels der Rotationssensoreinheit 35 eine Orientierung und/oder Lage und/oder Ausrichtung der Zubehöreinheit 30 im Raum erfasst. Die Rotationssensoreinheit 35 kann beispielsweise einen Gyroskop-Sensor zur Erfassung der Orientierung und/oder Lage und/oder Ausrichtung der Zubehöreinheit 30 umfassen.

Des Weiteren kann das Erfassen der Ist-Position der Zubehöreinheit 30 dabei mittels der Fixiereinheit 36 erfolgen. Beispielsweise kann mittels der Fixiereinheit 36 ein Fixierzustand der Zubehöreinheit 30 ermittelt werden. Hierbei kann es auch vorgesehen sein, dass der Fixierzustand mittels der Kameraeinheit erfasst wird, beispielsweise anhand einer Lage und/oder Position von Fixierbändern der Fixiereinheit 36. Des Weiteren kann der Fixierzustand auch anhand von direkt an der lokalen Hochfrequenzantenneneinheit33 angeordneter Fixierelemente bestimmt werden, indem beispielsweise anhand eines Steckzustands des Fixierelements der lokalen Hochfrequenzantenneneinheit 33 ein Fixierstatus und/oder ein Fixierzustand der Zubehöreinheit 30 ermittelt werden kann.

Das Erfassen des Ist-Zustands der Zubehöreinheit 30 kann dabei ausschließlich mittels des Sensor-basierten Erfassens der Ist-Position anhand von Referenzpunkten erfolgen. Zudem kann das Erfassen des Ist-Zustands der Zubehöreinheit 30 auch ausschließlich mittels des Erfassens einer Orientierung der Zubehöreinheit 30 im Raum erfolgen. Zudem kann das Erfassen des Ist-Zustands der Zubehöreinheit 30 auch ausschließlich mittels des Erfassens des Fixierstatus der Zubehöreinheit 30 erfolgen. Zudem kann auch das Erfassen der Ist-Position der Zubehöreinheit 30 mittels des Sensor-basierten Erfassens der Ist-Position anhand von Referenzpunkten und/oder mittels des Erfassens einer Orientierung der Zubehöreinheit 30 im Raum und/oder mittels des Erfassens des Fixierstatus der Zubehöreinheit 30 erfolgen.

In einem weiteren Verfahrensschritt 101 erfolgt ein Ermitteln einer Soll-Position der Zubehöreinheit 30 mittels der Ermittlungseinheit 32. Zur Ermittlung der Soll-Position der Zubehöreinheit 30 wird von der Ermittlungseinheit 32 eine Untersuchungsregion des Untersuchungsobjekts 15, insbesondere des Patienten, von der Ermittlungseinheit 32 bestimmt. Dies kann beispielsweis anhand einer Untersuchungsinformation und/oder einer Patienteninformation erfolgen. Die Untersuchungsinformation und/oder die Patienteninformation können dabei innerhalb der Steuereinheit 22 hinterlegt sein und von der Ermittlungseinheit 32 abgerufen werden. Zudem können die Untersuchungsinformation und/oder die Patienteninformation auch in einem Krankenhaus-Informations-System (HIS) und/oder in einem Radiologie-Informations-System (RIS) hinterlegt sein und von der Ermittlungseinheit 32 abgerufen werden.

Des Weiteren werden zur Ermittlung der Soll-Position der Zubehöreinheit 30 Bilddaten einer Übersichtsmessung und/oder einer Localizer-Messung des Patienten zur Verfügung gestellt. Vorzugsweise wird die Übersichtsmessung und/oder Localizer-Messung mittels der Scannereinheit 11 der Magnetresonanzvorrichtung 10 durchgeführt. Anhand der Daten, insbesondere der Bilddaten, Übersichtsmessung kann ein Model des Patienten mittels der Ermittlungseinheit 32 erstellt werden. Vorzugsweise wird das Model an die Bilddaten der Übersichtsmessung und/oder der Localizer-Messung von der Ermittlungseinheit 32 angefittet. Mittels des Models, das beispielsweise auch ein Avatar des Untersuchungsobjekts 15, insbesondere des Patienten, sein kann, des Patienten, kann somit sehr genau auf die Untersuchungsregion des Patienten und damit die Soll-Position der Zubehöreinheit 30 geschlossen werden, wie beispielsweise ein Lungenbereich des Patienten.

Sofern die Soll-Position der Zubehöreinheit 30 aufgrund eines vorherigen Ablaufen des Verfahrens zu einem Unterstützen des Benutzers bei einem Positionieren der Zubehöreinheit 30 für eine Magnetresonanzuntersuchung an dem Untersuchungsobjekt 15 bereits ermittelt wurde und vorliegt, kann das Ermitteln der Soll-Position der Zubehöreinheit 30 auch ein Abrufen der bereits ermittelten Soll-Position der Zubehöreinheit 30 umfassen.

In einem weiteren, daran anschließenden Verfahrensschritt 102 erfolgt ein Vergleichen der Ist-Position der Zubehöreinheit 30 mit der Soll-Position der Zubehöreinheit 30 hinsichtlich einer Abweichung der Ist-Position der Zubehöreinheit 30 von der Soll-Position der Zubehöreinheit 30. Die Abweichung zwischen der Ist-Position der Zubehöreinheit 30 und der Soll-Position der Zubehöreinheit 30 erfolgt hierbei sowohl hinsichtlich einer Position der Zubehöreinheit 30 als auch hinsichtlich einer Orientierung und/oder Lage und/oder Ausrichtung der Zubehöreinheit 30. Der Vergleich der Ist-Position der Zubehöreinheit 30 mit der Soll-Position der Zubehöreinheit 30 erfolgt mittels der Ermittlungseinheit 32.

In einem weiteren Verfahrensschritt 103 wird von der Ermittlungseinheit 32 eine erste Ausgabeinformation anhand des Vergleichs der Ist-Position der Zubehöreinheit 30 mit der Soll-Position der Zubehöreinheit 30 generiert. Die erste Ausgabeinformation enthält eine Information für den Benutzer, wie gut die Ist-Position der Zubehöreinheit 30 mit der Soll-Position der Zubehöreinheit 30 übereinstimmt. Zudem kann die erste Ausgabeinformation auch eine Positionsinformation der Soll-Position der Zubehöreinheit 30 umfassen.

In einem weiteren Verfahrensschritt 104 erfolgt die Ausgabe der ersten Ausgabeinformation an den Benutzer mittels der Ausgabeeinheit 29 der zweiten Benutzerschnittstelle 28.

Sofern die Ist-Position der Zubehöreinheit 30 mit der Soll-Position der Zubehöreinheit 30 übereinstimmt, kann eine weitere Ausgabeinformation generiert werden. Diese weitere Ausgabeinformation kann beispielsweise eine Aufforderung an den Benutzer umfassen, die Zubehöreinheit 30, insbesondere die lokale Hochfrequenzantenneneinheit 33, zu fixieren. Mittels der Ausgabeeinheit 29 kann anschließend die weitere Ausgabeinformation ebenfalls ausgegeben.

Sofern die Ist-Position der Zubehöreinheit 30 von der Soll-Position der Zubehöreinheit 30 abweicht, wird von der Ermittlungseinheit 32 eine Positionskorrektur der Ist-Position der Zubehöreinheit 30 in einem weiteren Verfahrensschritt 105 ermittelt. Die Positionskorrektur der Ist-Position umfasst bevorzugt einen Korrekturwert, so dass die Ist-Position der Zubehöreinheit 30 unter Berücksichtigung des Korrekturwertes mit der Soll-Position der Zubehöreinheit 30 übereinstimmt. Anhand der Positionskorrektur der Ist-Position der Zubehöreinheit 30 wird in einem weiteren Verfahrensschritt 106 eine zweite Ausgabeinformation generiert, die die Positionskorrektur der Ist-Position der Zubehöreinheit 30 umfasst. Die zweite Ausgabeinformation umfasst bevorzugt einen Korrekturvorschlag für die Zubehöreinheit 30 an den Benutzer. In einem weiteren Verfahrensschritt 107 wird die Ausgabeinformation an den Benutzer ausgegeben mittels der zweiten Benutzerschnittstelle 28, insbesondere der Ausgabeeinheit 29 der Benutzerschnittstelle 28.

Anschließend erfolgt in einem weiteren Verfahrensschritt 108 ein Positionieren der Zubehöreinheit 30, insbesondere der lokalen Hochfrequenzantenneneinheit 33, an dem Patienten.

Sobald Zubehöreinheit 30 neu positioniert ist, erfolgt ein erneutes Erfassen der Ist-Position der Zubehöreinheit 30 und die Verfahrensschritte 100 bis 104 werden erneut automatisch und/oder selbsttätig mittels der Ermittlungseinheit 32 durchgeführt. Erst wenn die Ist-Position der Zubehöreinheit 30 mit der Soll-Position der Zubehöreinheit 30 übereinstimmt, wird dem Benutzer dies durch die Ausgabeeinheit 29 und der weiteren Ausgabeinformation in dem Verfahrensschritt 104 signalisiert.

Sobald die Zubehöreinheit 30, insbesondere die lokale Hochfrequenzantenneneinheit 33, an der korrekten Position mit der korrekten Orientierung und/oder Lage angeordnet ist, wird dies bei einem Durchlaufen des erfindungsgemäßen Verfahrens in dem Verfahrensschritt 102 erkannt und in den Verfahrensschritten 103 und 104 eine entsprechende Ausgabeinformation generiert und an den Benutzer ausgegeben. Zudem kann in einem weiteren, optionalen Verfahrensschritt 110 eine weitere Ausgabeinformation von der Ermittlungseinheit 32 generiert werden. Diese weitere Ausgabeinformation kann beispielsweise eine Aufforderung zu einem Fixieren der Zubehöreinheit 30, insbesondere der lokalen Hochfrequenzspuleneinheit 33, in der aktuellen Ist-Position der Zubehöreinheit 30 umfassen. Anschließend kann in einem weiteren Verfahrensschritt 111 diese weitere Ausgabeinformation an den Benutzer, insbesondere an das medizinische Bedienpersonal, mittels der Ausgabeeinheit 29 der zweiten Benutzerschnittstelle 28 ausgegeben werden. Zudem kann in einem optionalen Verfahrensschritt 109 auch die Magnetresonanzuntersuchung an dem Patienten gestartet werden.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zu einem Unterstützen eines Benutzers bei einem Positionieren einer Zubehöreinheit (30) für eine Magnetresonanzuntersuchung an einem Untersuchungsobjekt (15) mit den folgenden Schritten:
- Erfassen einer Ist-Position der Zubehöreinheit (30) mittels einer Erfassungseinheit (31),
- Ermitteln einer Soll-Position der Zubehöreinheit (30) mittels einer Ermittlungseinheit (32),
- Vergleichen der Ist-Position der Zubehöreinheit (30) mit der Soll-Position der Zubehöreinheit (30) hinsichtlich einer Abweichung der Ist-Position der Zubehöreinheit (30) von der Soll-Position der Zubehöreinheit (30),
- Generieren einer ersten Ausgabeinformation anhand des Vergleichs der Ist-Position der Zubehöreinheit (30) mit der Soll-Position der Zubehöreinheit (30) und
- Ausgabe der ersten Ausgabeinformation an einen Benutzer mittels einer Ausgabeeinheit (29).

2. Verfahren nach Anspruch 1, umfassend die Schritte:
- Ermitteln einer Positionskorrektur der Ist-Position der Zubehöreinheit (30), sofern eine Abweichung der Ist-Position der Zubehöreinheit (30) von der Soll-Position der Zubehöreinheit (30) vorliegt, mittels der Ermittlungseinheit (32),
- Generieren einer zweiten Ausgabeinformation umfassend die Positionskorrektur der Ist-Position der Zubehöreinheit (30),
- Ausgabe der zweiten Ausgabeinformation an einen Benutzer mittels der Ausgabeeinheit (29).

3. Verfahren nach einem der vorhergehenden Ansprüche, wobei die Zubehöreinheit (30) eine an dem Untersuchungsobjekt (15) anliegende Hochfrequenzantenneneinheit (33) zur Erfassung von Magnetresonanzsignalen umfasst.

4. Verfahren nach einem der vorhergehenden Ansprüche, wobei das Erfassen der Ist-Position der Zubehöreinheit (30) ein Sensor-basiertes Erfassen der Ist-Position der Zubehöreinheit (30) umfasst, wobei das Sensor-basierte Erfassen der Ist-Position der Zubehöreinheit (30) anhand von Referenzpunkten des Untersuchungsobjekts (15) und/oder anhand von Referenzpunkten der Magnetresonanzvorrichtung (10) erfolgt.

5. Verfahren nach einem der vorhergehenden Ansprüche, wobei das Erfassen der Ist-Position der Zubehöreinheit (30) ein Erfassen einer Orientierung der Zubehöreinheit (30) im Raum umfasst.

6. Verfahren nach einem der vorhergehenden Ansprüche, wobei das Erfassen der Ist-Position der Zubehöreinheit (30) ein Erfassen eines Fixierungsstatus der Zubehöreinheit (30) umfasst.

7. Verfahren nach einem der vorhergehenden Ansprüche, wobei das Ermitteln der Soll-Position der Zubehöreinheit (30) ein Ermitteln einer Untersuchungsregion des Untersuchungsobjekts (15) umfasst.

8. Verfahren nach einem der vorhergehenden Ansprüche, wobei das Ermitteln der Soll-Position der Zubehöreinheit (30) ein Ermitteln anhand einer Übersichtsmessung des Untersuchungsobjekts (15) umfasst.

9. Verfahren nach einem der vorhergehenden Ansprüche, wobei zur Bestimmung der Soll-Position der Zubehöreinheit (30) ein Model des Untersuchungsobjekts (15) erstellt wird.

10. Verfahren nach einem der vorhergehenden Ansprüche, wobei die erste Ausgabeinformation eine Positionierungsinformation für die Soll-Position der Zubehöreinheit (30) umfasst.

11. Verfahren nach einem der vorhergehenden Ansprüche, wobei eine weitere Ausgabeinformation generiert wird, sobald die Ist-Position der Zubehöreinheit (30) mit der Soll-Position der Zubehöreinheit (30) übereinstimmt.

12. Verfahren nach einem der vorhergehenden Ansprüche, wobei die weitere Ausgabeinformation ausgegeben wird.

13. Magnetresonanzvorrichtung (10) mit einer Scannereinheit (11), einer Erfassungseinheit (31)zu einem Erfassen einer Ist-Position einer Zubehöreinheit (30), einer Ermittlungseinheit (32) zu einem Ermitteln einer Soll-Position der Zubehöreinheit (30), einer Ausgabeeinheit (29) und einer Zubehöreinheit (30), wobei die Magnetresonanzvorrichtung (10) zu einem Ausführen des Verfahrens zu einem Unterstützen eines Benutzers bei einem Positionieren einer Zubehöreinheit (30) für eine Magnetresonanzuntersuchung an einem Untersuchungsobjekt (15) nach einem der Ansprüche 1 bis 12 ausgelegt ist.

14. Magnetresonanzvorrichtung (10) nach Anspruch 13, **dadurch gekennzeichnet, dass** die Erfassungseinheit (31)eine Sensoreinheit (34) umfasst.

15. Magnetresonanzvorrichtung (10) nach einem der Ansprüche 13 bis 14,
**dadurch gekennzeichnet, dass** die Erfassungseinheit (31)eine Rotationssensoreinheit (35) umfasst.

16. Magnetresonanzvorrichtung (10) nach einem der Ansprüche 13 bis 15,
**dadurch gekennzeichnet, dass** die Erfassungseinheit (31)eine Fixiereinheit (36) umfasst.

17. Magnetresonanzvorrichtung (10) nach einem der Ansprüche 13 bis 16,
**dadurch gekennzeichnet, dass** die Zubehöreinheit (30) eine an dem Untersuchungsobjekt (15) anliegende Hochfrequenzantenneneinheit (33) zur Erfassung von Magnetresonanzsignalen umfasst.

18. Magnetresonanzvorrichtung (10) nach einem der Ansprüche 13 bis 17,
**dadurch gekennzeichnet, dass** die Ausgabeeinheit (29) an der Scannereinheit (11) und/oder der Zubehöreinheit (30) angeordnet ist.

19. Computerprogrammprodukt, welches ein Programm umfasst und direkt in einem Speicher einer programmierbaren Steuereinheit (22) einer Magnetresonanzvorrichtung (10) ladbar ist, mit Programmmitteln, um ein Verfahren zu einem Unterstützen eines Benutzers bei einem Positionieren einer Zubehöreinheit (30) für eine Magnetresonanzuntersuchung an einem Untersuchungsobjekt (15) nach einem der Ansprüche 1 bis 12 auszuführen, wenn das Programm in der Steuereinheit (22) der Magnetresonanzvorrichtung (10) ausgeführt wird.

20. Elektronisch lesbarer Datenträger mit darauf gespeicherten elektronisch lesbaren Steuerinformationen, welche derart ausgestaltet sind, dass sie bei Verwendung des Datenträgers in einer Steuereinheit (22) einer Magnetresonanzvorrichtung (10) das Verfahren zu einem Unterstützen eines Benutzers bei einem Positionieren einer Zubehöreinheit (30) für eine Magnetresonanzuntersuchung an einem Untersuchungsobjekt (15) nach einem der Ansprüche 1 bis 12 durchführen.
